(19) Europäisches Patentamt
European Patent Office
Office européen des brevets

(11) Publication number: **0 199 485 B1**

(12) **EUROPEAN PATENT SPECIFICATION**

(45) Date of publication of patent specification: **30.10.91**

(51) Int. Cl.5: **C07D 241/08**, C07D 405/06, C07D 409/06, C07D 487/04, C07D 498/04

(21) Application number: **86302548.2**

(22) Date of filing: **07.04.86**

The file contains technical information submitted after the application was filed and not included in this specification

(54) **Intermediates and process.**

(30) Priority: **12.04.85 GB 8509435**

(43) Date of publication of application: **29.10.86 Bulletin 86/44**

(45) Publication of the grant of the patent: **30.10.91 Bulletin 91/44**

(84) Designated Contracting States: **BE CH DE FR GB IT LI NL**

(56) References cited:
**EP-A- 0 086 678**
**EP-A- 0 134 984**

(73) Proprietor: **BEECHAM GROUP PLC**
**Beecham House Great West Road**
**Brentford Middlesex TW8 9BD(GB)**

(72) Inventor: **Dorgan, Roderick John**
**Lingworth, Scotts Hill**
**Outwood Surrey, RH1 5PR(GB)**
Inventor: **Elliot, Richard Leonard**
**18 Little Bookham Street Great Bookham**
**Leatherhead Surrey, KT23 3AQ(GB)**
Inventor: **Webster, Richard Andrew Bentley**
**86 Surrey Grove**
**Sutton Surrey, SM1 3PN(GB)**

(74) Representative: **Lockwood, Barbara Ann et al**
**Beecham Pharmaceuticals Biosciences Research Centre Great Burgh Yew Tree Bottom Road**
**Epsom Surrey KT18 5XQ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to piperazine derivatives useful as intermediates in the preparation of certain benzazepines and to a process for the preparation of such intermediates.

Our copending European Application Publication No. 0134984 discloses compounds of a general formula (A):

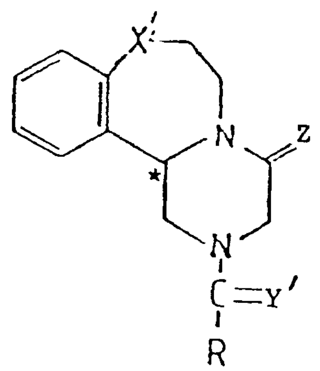

(A)

in which R is optionally substituted phenyl; $C_{3-8}$ cycloalkyl; $C_{5-8}$ cycloalkenyl; $C_{1-8}$ alkyl which may be straight or branched; $C_{2-8}$ alkenyl which may be straight or branched; 5- or 6- membered heterocyclyl; or optionally substituted phenyl $C_{1-4}$ alkyl, each of Y' and Z, which may be the same or different, is oxygen or sulphur; and X' is $-CH_2-$ or oxygen, and a process for the preparation of such compounds.

EP 0134984 A also discloses compounds of a general formula (B):

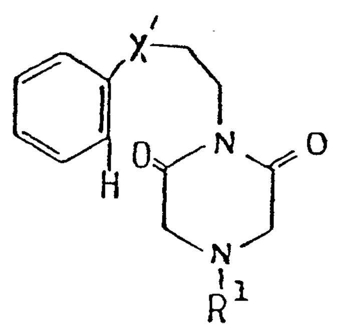

(B)

wherein X', is as defined in relation to formula (A) and $R^1$ is hydrogen, a protecting group or a group

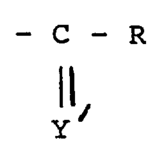

wherein R and Y' are as defined in relation to formula (A), useful as intermediates in the preparation of the compounds of formula (A).

We have now discovered that a certain class of the compounds of formula (B) are particularly useful as intermediates for the preparation of the corresponding benzazepine compounds.

Accordingly, the present invention provides a compound of formula (I):

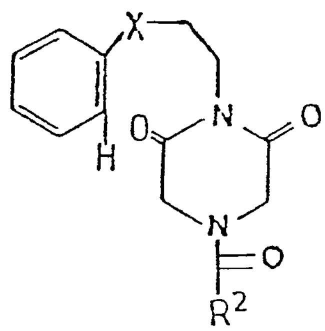

(I)

wherein $R^2$ is unsubstituted or substituted phenyl or $C_{3-8}$ cycloalkyl, $C_{5-8}$ cycloalkenyl, pyranyl or thiopyranyl and X is $-CH_2$-or oxygen.

When $R^2$ is substituted phenyl, it is substituted with one or more moieties selected from halogen, $C_{1-6}$ alkyl, $C_{1-6}$ alkoxy, nitro, amino, mono-or-di-$C_{1-6}$ alkylamino, and hydroxy.

A preferred $C_{3-8}$ cycloalkyl group is cyclohexyl.

In a particularly preferred aspect the present invention provides 4-(cyclohexylcarbonyl)-1-(3-phenylpropyl)piperazine-2,6-dione.

In an alternative aspect the present invention provides a process for the preparation of the hereinbefore defined compound of formula (I), which process comprises:

a) reacting a compound of formula (II):

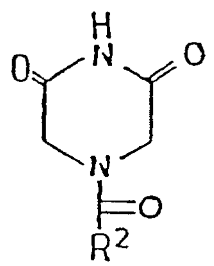

(II)

or a salt thereof, wherein $R^2$ is as defined in relation to formula (I), with a compound of formula (III):

$C_6H_5$-X-$(CH_2)_2$-$R^3$ (III)

wherein $R^3$ is a leaving group and X is as defined in relation to formula (I); or

b) reacting a compound of formula (IV):

$R^2$CO.Y (IV)

wherein $R^2$ is as defined in relation to formula (I) and Y is a leaving group, with 1-(3-phenylpropyl) piperazine-2,6-dione.

Suitably, $R^3$ is a bromine or iodine atom or a tosylate group, preferably an iodine atom.

Suitably, Y is a halogen atom.

Preferably, Y is a chlorine atom.

Suitably, the compound of formula (III) is reacted with an alkali metal salt, preferably the potassium or sodium salt, of the compound of formula (II).

Suitably the alkali metal salt of the compound of formula (II) is formed in situ by reacting the compound of formula (II) with an alkali metal salt much as potassium carbonate, preferably finely divided. Sodium hydride may be used for small scale aplications.

Suitably, the reaction between compounds of formula (II) and (III) is carried out in an aprotic solvent, for example dimethylformamide, at ambient to elevated temperatures until complete, within a temperature range of from 30°C to 120°C, preferably 70°C to 110°C.

Suitably, the reaction between compounds (IV) and 1-(3-phenylpropyl)piperazine-2,6-dione is carried out in an inert solvent, for example dichloromethane or chloroform, in the presence of a suitable base such as

triethylamine at any convenient temperature for example 0 to 5°C.

The reaction may be monitored by conventional techniques such as thin layer chromatography. On completion the required product may be isolated and purified by conventional techniques such as preparative chromatography.

The intermediate compounds of the invention may then be converted into compounds of the general formula (A) by methods disclosed by analogy in EP 0134984.

More particularly, the invention provides a process for the preparation of a compound of formula (V).

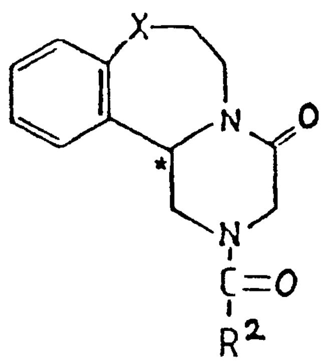

(V)

wherein $R^2$ and X are as defined in relation to formula (I), which process comprises cyclising a compound of formula (I).

Compounds of formula (I) may be cyclised by treatment with an acid catalyst, and conveniently an acid such as concentrated sulphuric acid may be used, in which case the reaction is carried out at a low temperature , for example from -10°C to 40°C and preferably less than 25°, more particularly between 0° and 5°C .

Suitably, the compound of formula (I) is dissolved in the minimum amount of an inert solvent such as dichloromethane or, more particularly, chloroform, prior to addition to the acid catalyst.

The following Examples illustrate the invention.

Preparation 1X

1-(3-Phenylpropyl)piperazine-2,6-dione

A solution of 1-(3-phenylpropyl)-4-benzyl-piperazine-2,6-dione (3.0 g) in a mixture of acetic acid (80 ml) and water (8 ml) was hydrogenated at 45°C and atmospheric pressure in the presence of a palladium on charcoal catalyst (0.65 g) for a period of 3 h. The mixture was filtered through celite, the solvents removed in vacuo and the residue dissolved in 5% aqueous hydrochloric acid. This solution was washed with chloroform, basified by addition of solid sodium carbonate, extracted with chloroform and the extract dried ($MgSO_4$). Evaporation of the solvent gave the title compound as a white solid 1.9 g (88%).

Example 1

4-(Cyclohexylcarbonyl)-1-(3-phenylpropyl)piperazine-2,6 -dione

Finely divided potassium carbonate (11.40 kg, 82.5 moles) was added to a stirred solution of 4-(cyclohexylcarbonyl)piperazine-2,6-dione (6.167 kg, 27.5 moles) in dimethylformamide (33.5 $dm^3$) at ambient temperature and the stirred mixture heated at 60 to 65°C for 1 hour. 1-Bromo-3-phenylpropane (5.475 kg, 4.18 $dm^3$, 27.5 moles) and potassium iodide (4.93 kg, 29.7 moles) were added and the resultant mixture stirred and heated at 98 to 100°C for 45 minutes.

The mixture was cooled to 20°C, water (123 $dm^3$) was added, and the mixture was then extracted with ethyl acetate (2 x 72 $dm^3$). The extracts were combined, washed with water (2 x 98 $dm^3$) and dried over magnesium sulphate. After filtration, evaporation of the solvent to low volume (ca. 12 $dm^3$) caused

precipitation of the white product which was collected by filtration.

A second crop of material was isolated by evaporation of the filtrate and trituration of the obtained solid with isopropyl ethyl acetate.

Total yield 7.76 kg (82.4%),m.p. 89°C.

Example 2

4-(Cyclohexylcarbonyl)-1-(3-phenylpropyl) piperazine-2,6-dione

Cyclohexanoyl chloride (1.2 g) was added to a solution of 1-(3-phenylpropyl)piperazine-2,6-dione (1.85 g) in chloroform (70 ml, ethanol-free) maintained at 0°C and triethylamine (1.02 g) added. The mixture was maintained at 0°C for 30 minutes then at room temperature for 16 h. The solution was washed successively with dilute hydrochloric acid, saturated sodium bicarbonate and water. The solution was dried ($MgSO_4$) and evaporated to give the title compound as a white solid 2.7 g (100%).

Examples 3 and 4 demonstrate the preparation of 2-(cyclohexylcarbonyl)-4-oxo-1,2,3,4,6,7,8,12b-octahydropyrazino [2,1-a][2]benzazepine from the intermediate 4-(cyclohexylcarbonyl)-1-(3-phenylpropyl) piperazine-2,6-dione

Example 3

4-(Cyclohexylcarbonyl)-1-(3-phenylpropyl)-6-hydroxypiperazine-2-one

The piperazinedione from Example 1 (4.8 kg, 14.02 moles) was dissolved in dichloromethane (14.1 $dm^3$), ethanol (84.4 $dm^3$) was added and the solution was cooled to 0°C. A solution of copper (II) chloride dihydrate (2.629 kg, 15.42 moles) in ethanol (8.5 $dm^3$) was added and the resultant solution stirred at 0°C for 1 hour. Sodium borohydride (2.651 kg, 70.1 moles) was then added portionwise over 1 hour with vigorous stirring. During the addition, the temperature was maintained at ca. 0°C but rose rapidly at one point to 10°C with profuse gas evolution. Upon complete addition, the dark mixture was stirred at 0°C for 1.5 hours.

Methanol (7.6 $dm^3$) was added and the mixture stirred for 30 minutes, warning to 12°C. Water (305 $dm^3$) was then cautiously added, followed by dichloromethane (55 $dm^3$). After filtration through Celite and subsequent washing of the filter bed with dichloromethane (55 $dm^3$), the layers were separated and the aqueous phase extracted with dichloromethane (1 x 55 $dm^3$,1 x 40 $dm^3$).

The organic extracts were combined, washed with saturated sodium chloride solution (65 $dm^3$), dried ($MgSO_4$) and, after filtration, evaporated to a colourless oil. This was dissolved in warm (30°C) isopropyl alcohol (4.5 $dm^3$), and warm (50°C) isopropyl ether (45 $dm^3$) was added to precipitate 4.13 kg (85.5%) of the product as a white solid, m.p. 111°C.

Example 4

2-(Cyclohexylcarbonyl)-4-oxo-1,2,3,4,6,7,8,12b-octahydropyrazino-[2,1-a][2]benzazepine.

A solution of the hydroxypiperazine from Example 3 (4.03 kg, 11.7 moles) in chloroform (6.1 $dm^3$) was added over 1 hour to stirred concentrated sulphuric acid (22 $dm^3$) at 0°C. The resulting solution was stirred at 0°C for 2 hours, then poured slowly with caution onto crushed ice (ca. 150 $dm^3$) maintaining the temperature below 5°C. Chloroform (20 $dm^3$) was added, the layers separated and the aqueous phase extracted with chloroform (2 x 16 $dm^3$). The organic extracts were combined, washed with excess saturated sodium hydrogen carbonate solution (28 $dm^3$), saturated sodium chloride solution (28 $dm^3$), dried ($MgSO_4$) and evaporated to a white solid. Purification by recrystallisation from ethyl acetate (33 $dm^3$) and ethanol (10 $dm^3$) with hot filtration gave two crops of the title compound totalling 3.24 kg (84.8%), m.p. 189°C.

**Claims**

**1.** A compound of formula (I):

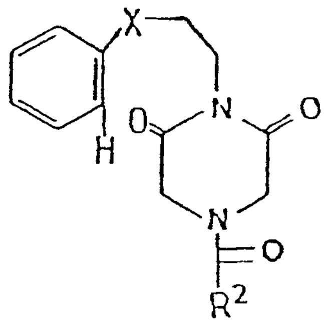

(I)

wherein $R^2$ is unsubstituted phenyl or phenyl substituted with one or more moieties selected from halogen, $C_{1-6}$ alkyl, $C_{1-6}$alkoxy, nitro, amino, mono-or-di-$C_{1-6}$ alkylamino and hydroxy, or $C_{3-8}$ cycloalkyl, $C_{5-8}$ cycloalkenyl,pyranyl or thiopyranyl and X is $-CH_2$-or oxygen.

2. A compound as claimed in claim 1, wherein $R^2$ is cyclohexyl.

3. A compound as claimed in claim 1, being 4-(cyclohexylcarbonyl)-1-(3-phenylpropyl) piperazine-2,6-dione.

4. A process for the preparation of a compound of formula (I), which process comprises reacting a compound of formula (II):

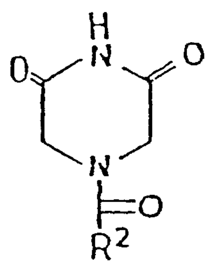

(II)

or a salt thereof, wherein $R^2$ is as defined in claim 1, with a compound of formula (III):

$C_6H_5$-X-$(CH_2)_2$-$R^3$ (III)

wherein $R^3$ is a leaving group and X is as defined in claim 1.

5. A process as claimed in claim 4 wherein the compound of formula (III) is reacted with an alkali metal salt of the compound of formula (II).

6. A process for the preparation of a compound of formula (I), which process comprises reacting a compound of formula (IV):

$R^2$CO.Y (IV)

wherein $R^2$ is as defined in claim 1 and Y is a leaving group, with 1-(3-phenylpropyl)piperazine-2,6-dione.

7. A process for the preparation of a compound of formula (V):

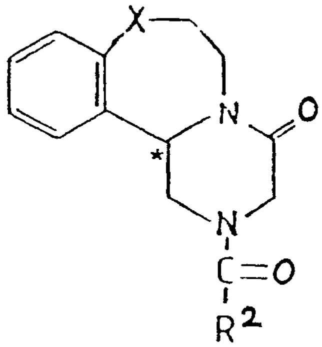

(V),

wherein $R^2$ and X are as defined in claim 1, which process comprises cyclizing a compound of formula (I) as defined in claim 1.

**8.** A process according to claim 7, wherein X is $-CH_2-$ and $R^2$ is cyclohexyl.

**9.** A process for the preparation of a compound of formula (V):

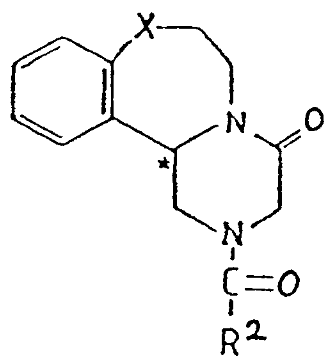

(V)

wherein $R^2$ is cyclohexyl and X is $CH_2$, which process comprises the steps of:
(i) reacting a compound of formula (IV):

$R^2CO.Y$ (IV)

wherein $R^2$ is cyclohexyl and Y is a leaving group, with 1-(3-phenylpropyl)piperazine-2,6-dione; or

reacting a compound of formula (II):

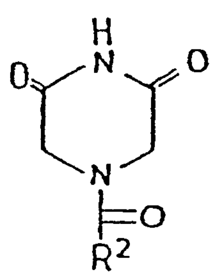

(II)

or a salt thereof, wherein $R^2$ is cyclohexyl with a compound of formula (III):

$C_6H_5-X-(CH_2)_2-R^3$ (III)

wherein $R^3$ is a leaving group and X is $CH_2$;

(ii) converting the intermediate of formula (I) produced in (i) to the corresponding 6-hydroxypiperazin-2-one by treatment with a suitable reducing agent; and
(iii) cyclizing the intermediate produced in step (ii).

## Revendications

**1.** Composé de formule (I) :

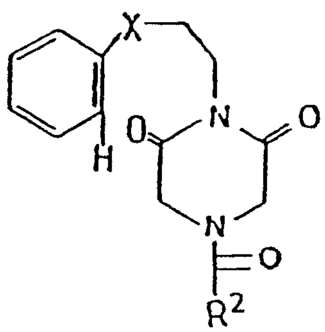

(I)

dans laquelle $R^2$ est un groupe phényle non-substitué ou phényle substitué avec un ou plusieurs éléments choisis parmi un atome d'halogène, un groupe alkyle en $C_{1-6}$ , alcoxy en $C_{1-6}$ , nitro, amino, mono- ou di-alkyl($C_{1-6}$)amino et hydroxy, ou un groupe cycloalkyle en $C_{3-8}$ , cycloalcényle en $C_{5-8}$ , pyranyle ou thiopyranyle et X est un radical $-CH_2-$ ou un atome d'oxygène.

**2.** Composé suivant la revendication 1, caractérisé en ce que $R^2$ est un groupe cyclohexyle.

**3.** Composé suivant la revendication 1, caractérisé en ce qu'il s'agit de la 4-(cyclohexylcarbonyl)-1-(3-phénylpropyl)pipérazine-2,6-dione.

**4.** Procédé pour la préparation d'un composé de formule (I), caractérisé en ce qu'il comprend la réaction d'un composé de formule (II) :

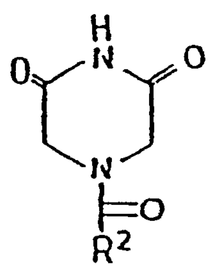

(II)

ou d'un sel de celui-ci, dans laquelle $R^2$ est tel que défini dans la revendication 1, avec un composé de formule (III) :

$C_6H_5-X-(CH_2)_2-R^3$ (III)

dans laquelle $R^3$ est un groupe mobile et X est tel que défini dans la revendication 1.

**5.** Procédé suivant la revendication 4, caractérisé en ce que le composé de formule (III) est traité avec un sel de métal alcalin d'un composé de formule (II).

**6.** Procédé pour la préparation d'un composé de formule (I), caractérisé en ce qu'il comprend la réaction d'un composé de formule (IV) :

$R^2CO.Y$ (IV)

dans laquelle $R^2$ est tel que défini dans la revendication 1 et Y est un groupe mobile, avec de la 1-(3-phénylpropyl)pipérazine-2,6-dione.

7. Procédé pour la préparation d'un composé de formule (V) :

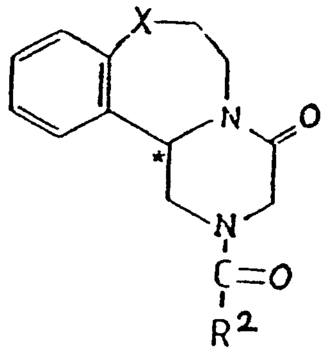

(V),

dans laquelle $R^2$ et X sont tels que définis dans la revendication 1, caractérisé en ce qu'il comprend la cyclisation d'un composé de formule (I) suivant la revendication 1.

8. Procédé suivant la revendication 7, caractérisé en ce que X est un radical $-CH_2-$ et que $R^2$ est un groupe cyclohexyle.

9. Procédé pour la préparation d'un composé de formule (V) :

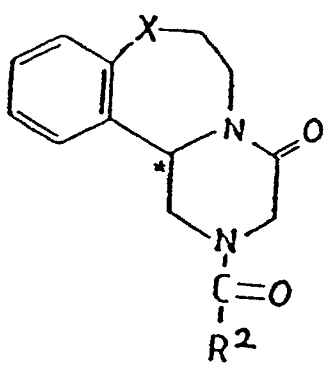

(V)

dans laquelle $R^2$ est un groupe cyclohexyle et X est un radical $-CH_2-$ , caractérisé en ce qu'il comprend les étapes suivantes :

(i) réaction d'un composé de formule (IV) :

$R^2CO.Y$ (IV)

dans laquelle $R^2$ est un groupe cyclohexyle et Y est un groupe mobile, avec la 1-(3-phénylpropyl)-pipérazine-2,6-dione ; ou

réaction d'un composé de formule (II) :

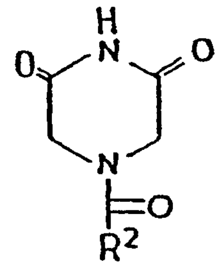

(II)

ou d'un sel de celui-ci dans laquelle $R^2$ est un groupe cyclohexyle avec un composé de formule (III) :

$C_6H_5$-X-$(CH_2)_2$-$R^3$ (III)

dans laquelle $R^3$ est un groupe mobile et X est un radical $-CH_2-$ ;
(ii) conversion de l'intermédiaire de formule (I) produit dans l'étape (i) en 6-hydroxypipérazin-2-one correspondante par traitement avec un agent réducteur convenable ; et
(iii) cyclisation de l'intermédiaire produit dans l'étape (ii).

**Patentansprüche**

1. Verbindung der Formel (I),

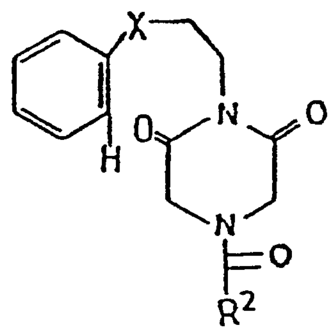

(I)

in der $R^2$ eine unsubstituierte Phenylgruppe, ein Phenylrest, der mit einer oder mehreren Einheiten gewählt aus einem Halogenatom, $C_{1-6}$-Alkylrest, $C_{1-6}$-Alkoxyrest,einer Nitrogruppe, einer Aminogruppe, einem Mono- oder Di-$C_{1-6}$-alkylaminorest und einer Hydroxygruppe substituiert ist, oder ein $C_{3-8}$-Cycloalkylrest, $C_{5-8}$-Cycloalkenylrest, eine Pyranyl- oder Thiopyranylgruppe und X $-CH_2-$ oder ein Sauerstoffatom ist.

2. Verbindung nach Anspruch 1, in der $R^2$ eine Cyclohexylgruppe ist.

3. Verbindung nach Anspruch 1, nämlich 4-(Cyclohexylcarbonyl)-1-(3-phenylpropyl)-piperazin-2,6-dion.

4. Verfahren zur Herstellung einer Verbindung der Formel (I), umfassend die Umsetzung einer Verbindung der Formel (II),

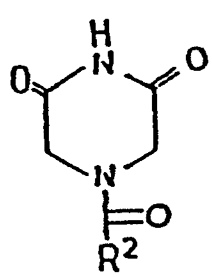

(II)

oder eines Salzes davon, wobei $R^2$ wie in Anspruch 1 definiert ist, mit einer Verbindung der Formel (III),

$C_6H_5$-X-$(CH_2)_2$-$R^3$ (III)

in der $R^3$ eine Abgangsgruppe ist und X wie in Anspruch 1 definiert ist.

5. Verfahren nach Anspruch 4, in dem die Verbindung der Formel (III) mit einem Alkalimetallsalz der Verbindung der Formel (II) umgesetzt wird.

6. Verfahren zur Herstellung einer Verbindung der Formel (I), umfassend die Umsetzung einer Verbindung der Formel (IV),

$R^2$CO.Y (IV)

in der $R^2$ wie in Anspruch 1 definiert und Y eine Abgangsgruppe ist, mit 1-(3-Phenylpropyl)piperazin-2,6-dion.

7. Verfahren zur Herstellung einer Verbindung der Formel (V),

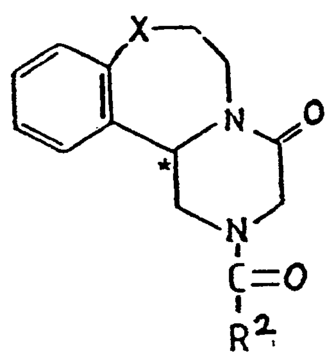

(V)

in der $R^2$ und X wie in Anspruch 1 definiert sind, umfassend eine Cyclisierung der Verbindung der Formel (I) wie in Anspruch 1 definiert.

8. Verfahren nach Anspruch 7, in dem X -$CH_2$- bedeutet und $R^2$ eine Cyclohexylgruppe ist.

9. Verfahren zur Herstellung einer Verbindung der Formel (V),

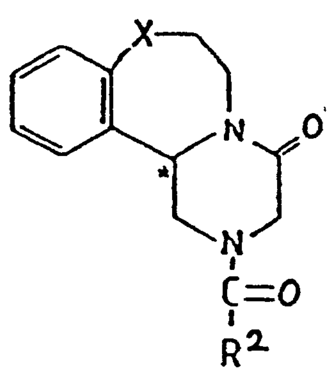

(V)

in der $R^2$ eine Cyclohexyl- und X eine $CH_2$-Gruppe ist, umfassend die Stufen:
(i) Umsetzen einer Verbindung der Formel IV;

$R^2$CO.Y (IV)

in der $R^2$ eine Cyclohexyl- und Y eine Abgangsgruppe ist, mit 1-(3-Phenylpropyl)-piperazin-2,6-dion;

oder

Umsetzen einer Verbindung der Formel (II)

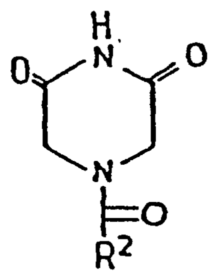

(II)

oder deren Salz, wobei $R^2$ eine Cyclohexylgruppe ist, mit einer Verbindung der Formel (III)

$C_6H_5$-X-$(CH_2)_2$-$R^3$ (III)

in der $R^3$ eine Abgangs- und X eine $CH_2$-Gruppe ist;
(ii) Umwandeln des in (i) hergestellten Zwischenprodukts der Formel (I) zum entsprechenden 6-Hydroxypiperazin-2-on mit einem geeigneten Reduktionsmittel; und
(iii) Zyklisieren des in Stufe (ii) hergestellten Zwischenprodukts